# EUROPEAN PATENT SPECIFICATION

(11) **EP 2 705 762 B1**
(45) Date of publication and mention of the grant of the patent: **24.05.2017**
(21) Application number: 11864889.8
(22) Date of filing: 03.05.2011
(51) Int. Cl.: A23L 33/105, A23L 21/20, A61K 35/64, A61K 36/73, A61P 5/26

(54) **BIOLOGICALLY ACTIVE DIETARY SUPPLEMENT FOR NORMALIZING THE ANDROGEN LEVEL IN MEN AND THE OVERALL CONDITION AND REDUCING OBESITY**
BIOLOGISCH AKTIVE NAHRUNGSERGÄNZUNG ZUR NORMALISIERUNG DES ANDROGENSPIEGELS BEI MÄNNERN UND DES GESAMTZUSTANDES SOWIE ZUR REDUZIERUNG VON ADIPOSITAS
ADDITIF BIOACTIF POUR ALIMENTS DESTINÉ À LA NORMALISATION DU NIVEAU D'ANDROGÈNES, À L'AMÉLIORATION DU BIEN-ÊTRE GÉNÉRAL CHEZ ET LA LUTTE CONTRE L'OBÉSITÉ CHEZ L'HOMME

(30) Priority: 04.05.2010 RU 2010117666
(43) Date of publication of application: 12.03.2014
(73) Proprietor: Obshestvo S Ogranichennoj Otvetstvennostju "PARAFARM", Penza 440023 (RU)
(72) Inventor: ELISTRATOV, Dmitry Gennadjevich, Penza 440060 (RU)
(74) Representative: Engel, Christoph Klaus
(86) International application number: PCT/RU2011/000294
(87) International publication number: WO 2012/150872

(56) References cited:
- EP-A1- 2 407 035
- EP-A2- 2 508 192
- EP-A2- 2 510 812
- BY-C- 9 012
- RU-C1- 2 233 666
- RU-C1- 2 351 354
- KHISMATULLINA N.Z. APITERAPIYA. PERM: MOBILE 2005, pages 95 - 96, 77-78, 158, 168, 209, XP008169097

## Description

The invention relates to food industry, namely to biologically active dietary supplements (BADS) used for normalization of hormonal profile and general health in men and produced based on natural ingredients.

A prior art search on this matter showed the existence of a large number of known herbal-based agents and promoting the normalization of hormonal profile and metabolic processes.

There is a known broad-spectrum treatment and prevention remedy containing propolis and milk thistle oil (Patent of Russian Federation No. 2175238, A 61 K 35/78). Due to the claimed combination of its ingredients, this biologically active supplement is effective in terms of its ergogenic action, stimulation of mental and physical activity and natural resistance of the body, and improvement of gastrointestinal, urinogenital and respiratory functions.

There is a known herbal-based dietary supplement used for prevention of pathological symptoms of climacteric syndrome (Patent of Russian Federation No. 2197975). This dietary supplement contains herbal constituents with hormone-like properties comprised by such groups of compounds as isoflavonoids, lignans and coumarins, and is intended for prevention of dyshormonal pathological symptoms of climacteric and post-climacteric syndrome in men and women. The drawback of this known dietary supplement is that it does not address the endocrine regulation in men, the disturbance of which largely determines the development of obesity in aging men, leading to a variety of health disorders.

There is a known biologically active product containing drone brood and used to maintain proper metabolism and nutrition of body tissues and organs in men (Drone Milk by Tentorium Apicompany - found on the Web: http://medovik.com/page mol 0.html, 25.01.2008) .

The drawback of this known product is its low effect on metabolic processes in men and lack of data in regard to endocrine system regulation in men.

Patent document RU 2 233 666 discloses the uses of drone breed-based tablets for general tonic, anabolic and accessory effects in paediatric and geriatric practice.

There is a known preventive remedy used for prevention of thyroid disorders and containing roots and rhizomes of white cinquefoil, or aerial portions of white cinquefoil, or their mixture (Patent of Russian Federation No. 2351334). The drawback of this known remedy is its low efficacy in normalizing the hormonal profile and general health in men.

It is the object of this, which is defined by the claim, to develop a dietary supplement to prevent obesity in men with partial age-related androgen deficiency, and to normalize their androgen levels.

The invention concerns a biologically active dietary supplement for use in the treatment of the human body by normalizing the androgen levels in men, improving general health in men, and reducing obesity in men, wherein the supplement comprises roots and rhizomes of white cinquefoil or aerial portions of white cinquefoil, or their mixture, and drone brood in the following proportion: drone brood - 20 wt% to 80 wt%. Said invention aims to expand the range of biologically active dietary supplements provide a wide range of beneficial effects in the human body and promote rehabilitation of the population.

To achieve this object, potentiating the beneficial therapeutic effect of white cinquefoil was proposed by supplementing the formulation with drone brood as a donator of entomological sex hormones such as prolactin, estradiol, progesterone and testosterone stimulating the androgen functions in men. Rich in vitamins and hormones which are not hormone substitutes, drone brood is effective in treating hormonal disturbances. It offers a possibility of harmonizing the functioning of all organs and systems in the human body.

The objective is achieved by proposing a biologically active dietary supplement to normalize androgen levels in men, improve general health and reduce obesity, characterized in that it contains roots and rhizomes of white cinquefoil or aerial parts of white cinquefoil, or their mixture, and also contains drone brood in the following proportion: drone brood - 20 wt% to 80 wt%.

A technical result of the claimed invention is the creation of a dietary supplement with an optimally selected proportion of ingredients, free of side effects and suitable to be most effectively used for normalization of metabolic processes and hormonal profile in men due to its bioactive properties.

The applicability of a combination of said ingredients is supported by the fact that combined use of agents of herbal and animal origin has been observed to potentiate the pharmacological activity of ingredients, because the effects exerted by constituents of white cinquefoil and drone brood occur by different pathways.

White cinquefoil normalizes metabolic processes in the body by normalizing the activity of the thyroid gland. Hypothyroid state causes a slowing down of the metabolic processes and, consequently, leads to obesity.

The effect of drone brood is provided by enzymes, amino acids, a complex of vitamins, macro- and micronutrients, hormones and growth factors stimulating the cell metabolism and boosting fat burning within the body.

This ensures the integrity and versatility of therapeutic effects in the human body and makes it possible to address both the disease (consequence) and its underlying cause.

Some causes of obesity in men are:
1. Androgen deficiency,
2. Hypothyroidism,
3. Disordered digestion.

Most often, the existing treatment methods involve hormone replacement therapy, which is the traditional allopathic approach to treatment. The risk of this method is determined by two factors: 1) hormone dosages should be carefully adjusted, 2) hormone replacement therapy suppresses the body's own production of hormones.

The claimed dietary supplement provides concurrent normalization of thyroid gland function (and, respectively, of hormones produced by it) and the level of androgens produced by testes, and regulates the digestive function through the action of chemical constituents of white cinquefoil.

Examples of the claimed BADS formulation:
Example 1:
   Cinquefoil roots - 20 wt%
   Drone brood - 80 wt%
Example 2:
   Cinquefoil leaves - 50 wt%
   Drone brood - 50 wt%
Example 3:
   Cinquefoil leaves - 80 wt%
   Drone brood - 20 wt%

During the implementation of the objective, we revealed that the resulting action of the supplement was significantly more potent than that of each of its ingredients taken alone, due to a synergistic effect.

A six-month trial of the dietary supplement on 20 volunteers showed a significant decrease in the weight of fat deposits, reduction of dyspnea, normalization of heart rate, increase in general sense of well-being, improved sleep, enhanced sexual performance and normalization of hormonal profile.

The action of the claimed dietary supplement was compared to that of its individual ingredients.

Thus, 20 male volunteers, within the period of several months, received the claimed dietary supplement formulated as per Example 1 (1^{st} group); another 20 subjects received the dietary supplement formulated as per Example 3 (2^{nd} group); the 3^{rd} group of 20 subjects received cinquefoil in the form of powdered leaves, and 4^{th} group used the preventive remedy based on drone brood (Drone Milk). Hormonal androgen levels, body weight and general health condition were monitored in each group.

The study showed an increase in androgen levels in 90% of the subjects of the 1^{st} group and in 85% of the subjects of the 2^{nd} group, while in the 3rd and 4^{th} groups the increase in androgen levels over the same period of time was 40%,; a decrease in fat deposits was observed in 70% of subjects of the 1^{st} group, 60% of subjects in the 2^{nd} group, 30% of subjects in the 4^{th} group; only 20% of volunteers in the 3^{rd} group experienced a decrease in body weight. Subjects of the 1^{st} and 2^{nd} groups reported a significant improvement in their general sense of well-being.

Therefore, the claimed supplement shows greater efficacy in normalization of metabolic processes and hormonal profile in men as compared to the separate use of each of its ingredients.

The efficacy of the dietary supplement is explained by the fact that concurrent regulation of two endocrine glands is more effective as compared to regulating either of these functions alone. A characteristic effect of thyroid hormones is their ability to stimulate the energy metabolism by enhancing the oxidizing processes in the mitochondria, increasing the oxygen consumption and carbon dioxide elimination and increasing the utilization of carbohydrates, fats and proteins, which leads to weight loss and intensive uptake of glucose from the bloodstream.

It should be noted that fat tissue is currently viewed by physicians as an endocrine organ, and its excess is believed to adversely affect not only the metabolic rate, but also the testosterone production in men. All obese men have a relative or absolute decrease in the secretion of testosterone, and treating obesity is impossible without normalizing this function. Therefore, the use of drone brood influences the androgenic function in men and resists the action of fat tissue. The thyroid does not affect the endocrine functions of fat tissue.

The claimed dietary supplement is produced in powder, pill or capsule form, and may also be produced in the form of aqueous alcoholic extract and dosage forms prepared based on such extract, namely: powder, pills and capsules.

Therefore, despite the fact that all ingredients in the composition of the claimed supplement are known in folk and traditional medicine, their combination in a single product is not known from the prior art; namely, the revealed synergistic effect made it possible to accomplish the objective and to achieve the claimed technical result of creating a dietary supplement with an optimally selected proportion of ingredients, free of side effects and effective in prevention of obesity in men and normalizing their androgen levels.

## Claims

1. A biologically active dietary supplement for use in the treatment of the human body by normalizing the androgen levels in men, improving general health in men, and reducing obesity in men, wherein the supplement comprises roots and rhizomes of white cinquefoil or aerial portions of white cinquefoil, or their mixture, and drone brood in the following proportion: drone brood 20 wt% to 80 wt%.

## Patentansprüche

1. Biologisch aktives Nahrungsergänzungsmittel zur Verwendung in der Behandlung des menschlichen Körpers durch Normalisierung des Androgenspiegels bei Männern, durch Verbesserung der allgemeinen Gesundheit von Männern und durch Verringerung der Fettleibigkeit von Männern, wobei das Nahrungsergänzungsmittel folgende Bestandteile umfasst: Wurzeln und Rhizome von weißem Fingerkraut oder oberirdische Pflanzenteile von weißem Fingerkraut oder deren Mischungen, und Drohnenbrut mit einem Anteil von 20 Gewichts-% bis 80 Gewichts-%.

## Revendications

1. Complément alimentaire biologiquement actif destiné à traiter l'organisme humain en normalisant les taux d'androgènes chez les hommes, améliorant ainsi l'état de santé général chez les hommes, et réduisant l'obésité chez les hommes, où le complément alimentaire comprend des racines et des rhizomes de potentille blanche ou des parties aériennes de potentille blanche ou un mélange des deux, et du couvain de faux bourdon dans la proportion suivante : 20 à 80 % en poids de couvain de faux bourdon.
